(19) Europäisches Patentamt
European Patent Office
Office européen des brevets

(11) EP 3 173 071 B2

(12) NEW EUROPEAN PATENT SPECIFICATION
After opposition procedure

(45) Date of publication and mention of the opposition decision:
**24.12.2025 Bulletin 2025/52**

(45) Mention of the grant of the patent:
**24.03.2021 Bulletin 2021/12**

(21) Application number: **16200848.6**

(22) Date of filing: **28.11.2016**

(51) International Patent Classification (IPC):
***A61K 9/00*** *(2006.01)* ***A61K 31/439*** *(2006.01)*
***A61K 47/10*** *(2017.01)* ***A61K 47/40*** *(2006.01)*
***A61P 1/08*** *(2006.01)*

(52) Cooperative Patent Classification (CPC):
**A61K 31/439; A61K 9/0014; A61K 47/10; A61K 47/40; A61P 1/08**

# (54) MAROPITANT FORMULATION

MAROPITANTFORMULIERUNG

FORMULATION DE MAROPITANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.11.2015 NL 2015865**

(43) Date of publication of application:
**31.05.2017 Bulletin 2017/22**

(73) Proprietor: **Le Vet B.V.**
**3421 TV Oudewater (NL)**

(72) Inventors:
- **BOEREN, Marinus Maria Martinus**
  **3421 TV OUDEWATER (NL)**
- **PARIDAANS, Rudolf Johannes**
  **3421 TV OUDEWATER (NL)**

(74) Representative: **Mewburn Ellis LLP**
**Aurora Building**
**Counterslip**
**Bristol BS1 6BX (GB)**

(56) References cited:
**WO-A1-2013/078500 WO-A2-2005/082416**

- **ANONYMOUS: "CVMP assessment report for Cerenia new route of administration (intravenous use) for the solution for injection (EMEA/V/C/000106/X/0023)", EMEA, 10 April 2015 (2015-04-10), pages 1/12 - 2/12, XP055357742, Retrieved from the Internet <URL:http://www.ema.europa.eu/docs/en_GB/document_library/EPAR_-_Assessment_Report_-_Variation/veterinary/000106/WC500189398.pdf> [retrieved on 20170322]**
- **WU Z ET AL: "Absorption and tissue tolerance of ricobendazole in the presence of hydroxypropyl-b-cyclodextrin following subcutaneous injection in sheep", INTERNATIONAL JOURNAL OF PHARMACEUTICS, ELSEVIER, AMSTERDAM, NL, vol. 397, no. 1-2, 15 September 2010 (2010-09-15), pages 96 - 102, XP027230643, ISSN: 0378-5173, [retrieved on 20100819]**

EP 3 173 071 B2

## Description

The present invention relates to a pharmaceutical composition comprising maropitant, a method for preparing the composition, the use of the composition as a medicament, and the use of the composition in the treatment of emesis in mammals, in particular cats and dogs.

Maropitant, a substituted quinuclidine having the chemical name (2S,3S)-(Diphenylmethyl)-N-[2-methoxy-5-(2-methyl-2-propanyl)benzyl]quinuclidin-3-amine, the systematic (IUPAC) name being (2S,3S)-*N*-(5-*tert*-Butyl-2-methoxybenzyl)-2-(diphenylmethyl)-1-azabicyclo[2.2.2]octan-3-amine, is a neurokinin (NK1) receptor antagonist that blocks the pharmacological action of substance P in the central nervous system (CNS). Maropitant is used in the prevention and treatment of vomiting in dogs and the treatment of vomiting in cats.

WO 2005/082416 describes the development of a pharmaceutical formulation of maropitant citrate, sulfobutyl ether β-cyclodextrin and meta-cresol. WO 2005/082416 shows that meta-cresol was the only preservative tested that was also stable on storage and had an acceptable injection site tolerance. Other preservatives such as benzoic acid, ethanol, propylene glycol and the combination of 0.5 % chlorobutanol and 0.5 % phenylethanol, were all shown to have poor injection site tolerance.

Although meta-cresol was identified as being the most suitable preservative by the inventors of WO 2005/082416, studies have since shown that injection of a pharmaceutical formulation of maropitant citrate, sulfobutyl ether β-cyclodextrin and meta-cresol leads to pain and adverse reactions in animal subjects, in particular cats. For example, pain on injection of maropitant citrate, sulfobutyl ether β-cyclodextrin and meta-cresol was observed in 75% of cats injected with 3 and 5 mg/kg, whereas in the control group injected placebo (saline) only 5% of cats registered a pain response.

There is therefore a need for an injectable solution of maropitant which causes less pain on injection.

It is an object of the present invention to provide an injectable maropitant composition that has an improved injection site tolerance.

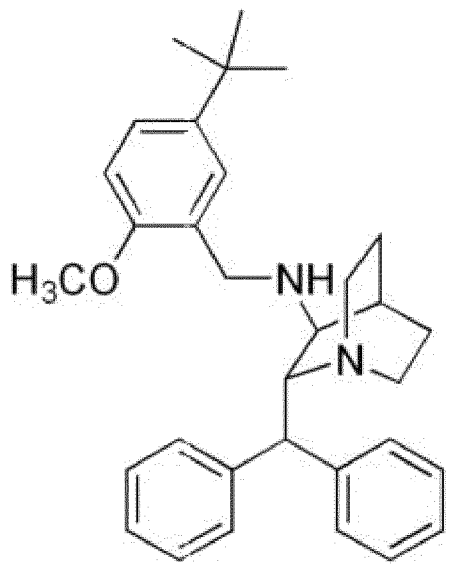

Formula I

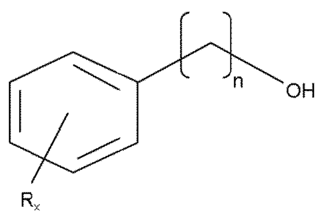

Formula II

The object of the present invention is solved by a pharmaceutical composition as defined in claim 1 and for use as defined in claim 9. The invention also relates to a method as defined in claim 12.

The inventors have found that injectable compositions according to the invention comprising phenylmethanol do not cause pain on injection in cats and dogs, in other words have an excellent injection site tolerance. Furthermore, phenylmethanol has antimicrobial effects when included in a composition comprising maropitant citrate and a pharmaceutically acceptable β-cyclodextrin.

The term "pharmaceutically acceptable" means that the substance or composition must be compatible chemically and toxicologically with the other ingredients of the composition and with the mammal being treated therewith.

By "injection site tolerance" is meant that when the composition according to the invention is injected into a subject, for example a cat, a low pain response is observed. Typically, pain response is determined by observing behavioural changes of the subject for example scratching, hissing, whining, startled look, flight response, desire to hide.

The pain score associated with the injection of a composition according to the present invention in cats leads to a modal score of 1 on a five-point pain scale (with a score of 1 indicating minimal or no pain response and a score of 5 indicating a severe pain response was observed). This is a considerable improvement over the commercially available composition of maropitant citrate (monohydrate), sulfobutyl ether β-cyclodextrin and meta-cresol which gives a modal score of 3 on the pain scale.

The inventors have, for the first time, been able to identify the cause of the pain response in the commercially used maropitant injectable formulations and have identified a composition which does not lead to pain on injection. The excellent acceptance of the injectable formulation according to the invention is surprising in light of the knowledge that in rats subcutaneous injection of benzyl alcohol leads to inflammatory responses (p.180, Water-insoluble Drug Formulation, 2nd Ed., Lui, Rong, CRC Press, Boca Raton, 2008). The inventors observe that, when phenylmethanol is used as preservative, a surprising lack of inflammatory and pain responses are observed on injection in cats and dogs, in particular when used in the claimed concentration of 7 - 18 mg/ml.

The inventors have found that inclusion of 7 - 18 mg/ml phenylmethanol as pharmaceutically acceptable preservative, results in the desired level of antimicrobial effects according to the requirements of Ph. Eur. A criteria and Ph. Eur. B criteria. Not only are the antimicrobial effects acceptable but the compositions of the present invention also have an acceptable and improved injection site tolerance.

In an attractive embodiment, the pharmaceutically acceptable preservative is free of chlorobutanol, preferably free of chlorinated compounds. Most preferably, the aqueous solution is free of chlorinated organic compounds, as such compounds would reduce the injection site tolerance. The inventors have found that by using at least 7 - 18 mg/ml phenylmethanol as pharmaceutically acceptable preservative, it is not necessary to include chlorobutanol to achieve the desired antimicrobial effects. Moreover, compositions according to the present invention show an improved injection site tolerance compared to compositions of the prior art which comprise chlorobutanol.

In an attractive embodiment, the pharmaceutically acceptable preservative is present at a concentration of 7-15 mg/ml, preferably 7-13 mg/ml, more preferably 8-12 mg/ml, even more preferably 9-11 mg/ml or even at about 10 mg/ml. The term 'about' means that 5% more or less is allowed, i.e. defining a range of 9.5-10.5 mg/ml. More preferably, the term 'about' means that 3% more or less is allowed, most preferably 1% or even 0%. Surprisingly, it has been found that concentrations of the pharmaceutically acceptable preservative of 10 mg/ml, and even up to 15 or even 17 or 18 mg/ml pharmaceutically acceptable preservative provides for the desirable osmolality, whilst showing good injection site tolerance.

It has been found that phenylmethanol which has a short hydrocarbyl group between the phenyl group and hydroxyl group is advantageous as a particularly low pain score in observed on injection into cats and dogs.

Pharmaceutically acceptable preservative being selected from the group consisting of phenylmethanol, 2-phenylethanol, 3-phenyl-1-propanol and 4-phenyl-1-butanol provide a composition that has a much better injection site tolerance than commercially available injectable maropitant compositions.

A particularly acceptable injection site tolerance is achieved by using a composition according to the invention comprising phenylmethanol. When the antimicrobial preservative is phenylmethanol, a pain score of 1 is observed, compared to a pain score of 3 using a commercially available composition comprising meta-cresol.

In another embodiment, the composition comprises 8-12 mg/ml maropitant, as calculated by the weight of the free base. In case a maropitant salt is used, the higher molecular weight of the salt should be corrected for. This would e.g. mean that a 8 mg/ml maropitant solution can be obtained by dissolving 8 mg maropitant free base (having a molecular weight of 469 g/mol) per ml of water, or e.g. 11.6 g maropitant citrate monohydrate (having a molecular weight of 679 g/mol). In an attractive embodiment, maropitant citrate is provided by combining 8-12 mg/ml maropitant free base and 2-6 mg/ml citric acid.

Preferably, maropitant free base is used in combination with a pharmaceutical grade citric acid solution. Alternatively, maropitant can be present as a pharmaceutically acceptable salt, for example citrate.

In the present application "cyclodextrin" means a compound including cyclic (is 1→4) linked D-glucopyranose units, α-cyclodextrin refers to a cyclodextrin with 6 cyclic, linked D-glucopyranose units, β-cyclodextrin has 7 cyclic, linked D-glucopyranose units, and γ-cyclodextrin has 8 cyclic, linked D-glucopyranose units. The cyclic D-glucopyranose units define a hydrophobic cavity. Due to the conformation of the glucopyranose units, the cyclodextrins are shaped like a

truncated cone rather than perfect cylinders. The hydroxyl functions are orientated to the cone exterior with the primary hydroxyl groups of the sugar residues at the narrow edge of the cone and the secondary hydroxyl groups at the wider edge. The central cavity is lined by the skeletal carbons and ethereal oxygens of the glucose residues, which gives it a lipophilic character.

Modifications to cyclodextrins can occur at one or more of the 2, 3 and 6 hydroxyl positions of one or more glucopyranose units. The sulfobutyl ether derivative of β-cyclodextrin (SBE-β-CD) has a range of six to seven sulfobutyl ether groups per cyclodextrin molecule Alternatively the hydroxyl groups can be modified by hydroxypropyl moieties resulting in hydroxylpropyl-β-cycodextrins (HP-β-CD)

In an embodiment, the pharmaceutically acceptable β-cyclodextrin is sulfobutyl ether β-cyclodextrin sodium or 2-hydroxypropyl β-cyclodextrin, preferably sulfobutyl ether β-cyclodextrin sodium.

It has been found that an acceptable injection site tolerance is observed when either sulfobutyl ether β-cyclodextrin sodium or 2-hydroxypropyl-β-cyclodextrin is used. For example, when the β-cyclodextrin is 2-hydroxypropyl-β-cyclodextrin, the observed modal pain score was 2 when a composition according to the present invention was subcutaneously injected into cats. This is an improvement on the commercially available composition, which has an observed modal pain score of 3.

It is particularly preferred to use sulfobutyl ether β-cyclodextrin sodium as the pharmaceutically acceptable cyclodextrin in the compositions according to the invention because when this composition was sub-cutaneously injected into cats the observed modal pain score was 1. The inventors have found that the combination of an aqueous solution of maropitant citrate, sulfobutyl ether β-cyclodextrin sodium and benzyl alcohol has a very acceptable injection site tolerance.

Furthermore the very low pKa of the sulfonic acid groups means that this modified cyclodextrin carries multiple negative charges at physiologically compatible pH values. Sulfobutyl ether β-cyclodextrin sodium is polyanionic in nature, and therefore interacts particularly well with maropitant. In addition, this sulfobutyl ether β-cyclodextrin sodium has exceptional solubility and parenteral safety.

Without wishing to be bound by theory, it is proposed that the low pain score associated with injection of compositions comprising sulfobutyl ether β-cyclodextrin is due to the osmolality of this composition being close to the physiological osmolality.

'Osmolality' is the concentration of a solution expressed as the total number of solute particles per kilogram. According to USP Pharmacists' Pharmacopoeia, Supplement, 2nd Edition, 1st December 2009, section 785, page S5/38, the osmolality of a solution ξm is given by:

$$\xi_m = \Sigma v_i m_i \Phi_{m,i}$$

where $v_i$ is the number of particles formed by the dissociation of one molecule of the $i^{lh}$ solute; $v_i = 1$ for nonionic (nondissociating) solutes;
mi is the molality of the $i^{th}$ solute; and
$\phi_{m,i}$ is the molal osmotic coefficient of the $i^{th}$ solute.

The osmolality of a real solution corresponds to the molality of an ideal solution containing non-dissociating solutes and is expressed in osmoles or milliosmoles per kilogram of solvent (Osmol per kg or mOsmol per kg, respectively), a unit that is similar to the molality of the solution. Thus, osmolality is a measure of the osmotic pressure exerted by a real solution across a semipermeable membrane.

It has been found that the osmolality of the composition according to the invention is between 290 and 310 mOsmol per kg. The inventors have found that by using 11 mg/ml phenylmethanol as the preservative, a composition having an osmolality of 301 mOsmol per kg is obtained, which is very close to the physiological osmolality (in cats and dogs) which is 300 mOsmol per kg. Similar osmolality values are obtained when using benzyl ethanol within he claimed range.

The effect of the close matching of the osmolality of the injected solution to that of the physiological osmolality means that the injected solution is effectively isotonic with the conditions of the animal receiving the injection, so that there is minimal physiological response in the said animal.

In contrast to the composition according to the present invention, the commercially available maropitant citrate monohydrate, sulfobutyl ether β-cyclodextrin and meta-cresol composition has an osmolality of about 214 mOsmol per kg. The inventors propose that this relatively low osmolality compared to the physiological osmolality is the cause of the high pain response in an animal. An osmolality quite significantly lower (i.e., more dilute) than the physiological osmolality will cause the red blood cells to take on extra fluid in an attempt to equilibrate osmolality on either side of the blood cell membrane. If they swell too much, they will burst, spilling their contents into the serum and, at least with the red blood cells, making them unavailable for oxygen transport, and thus causing pain for the recipient of the injection.

In another embodiment, the pharmaceutically acceptable β-cyclodextrin is present at a concentration of 40 - 80

mg/ml, preferably of 60 - 70 mg/ml. The inventors have found that inclusion of a β-cyclodextrin is essential for the solubility of the maropitant. The optimum concentration range has been found to be 60 - 70 mg/ml.

In an attractive embodiment, the aqueous solution is free of a co-solvent, such as ethanol, as such co-solvent will reduce the injection site tolerance.

In a second aspect of the present invention, the invention relates to a pharmaceutical composition for use as a medicament. The inventors have found that the composition according to the invention is clinically useful for the prevention and treatment of motion sickness and emesis in mammals and as an anti-nociceptive in mammals.

In a preferred embodiment, the pharmaceutical composition according to the invention is used in the treatment of emesis in mammals. In the context of the present invention, mammal includes animals such horse, sheep, goat, pig, cow, dog and cat.

In a particularly preferred embodiment, the pharmaceutical composition according to the invention for use in the treatment of emesis in cats and dogs. The present invention is particularly suited to the treatment of companion animals, such as cats and dogs.

In an embodiment, the pharmaceutical composition according to the invention is administered by parenteral injection. The compositions according to the invention are intended for administration as an injection or infusion. For example, the compositions may be administered intravenously (into a vein), subcutaneously (under the skin), and intramuscularly (into muscle). Preferably the compositions according to the present invention are administered subcutaneously. The present invention has the advantage that the composition according to the present invention has leads to a minimal, if any, pain response as observed on the said 5 point pain scale.

In a third aspect, the present invention relates to a method for preparing a pharmaceutical composition as defined above, comprising the steps of:

I) mixing, in an aqueous medium, the medium preferably being water:

- i) a source of maropitant,
- ii) a source of citrate,
- iii) a pharmaceutically acceptable β-cyclodextrin to the mixture, the pharmaceutically acceptable β-cyclodextrin preferably being sulfobutyl ether β-cyclodextrin sodium or 2-hydroxypropyl-β-cyclodextrin, most preferably sulfobutyl ether β-cyclodextrin,
- iv) a pharmaceutically acceptable preservative as defined in claim 1, to obtain an aqueous solution,

II) if the pH of the aqueous solution is outside the range of 3 - 6, adjusting the pH to between 3 and 6 by adding to the solution a pharmaceutically acceptable acid or base, the pharmaceutically acceptable base preferably being sodium hydroxide or ammonia, most preferably sodium hydroxide, the pharmaceutically acceptable acid preferably being selected from the group consisting of hydrochloric acid, formic acid, acetic acid and citric acid, preferably being citric acid.

The aqueous medium is preferably water, although any suitable a. The water can comprise additional solubles, that can e.g. be adjuvants in the solution. The skilled person is aware of suitable solubles that can be used in the solution. An aqueous medium can be used, which is preferably free of organic cosolvents.

Mixing the above compounds will result in dissolution thereof in the medium. The order of addition in the medium is not critical.

The inventors have found that the maropitant composition of the present invention can easily be prepared by providing an aqueous solution of a source of maropitant, for example as maropitant cations and citrate counter ions, and adding a suitable β-cyclodextrin to complex the maropitant cations and thus improve the solubility of the maropitant cations. Subsequently, the pharmaceutically acceptable preservative is added and the composition pH adjusted by titration with a pharmaceutically acceptable acid or base.

In an embodiment, the maropitant is provided by maropitant free base or a maropitant salt, said salt preferably being selected from the group consisting of hydrobromide, hydrochloride, hydroiodide, sulphate, acetate, malate, oxalate, lactate, citrate, fumerate, tartrate, mesylate and succinate. The inventors have found that both inorganic, for example hydrobromide, hydrochloride, hydroiodide and sulphate, as well as organic salts, for example acetate, malate, oxalate, lactate, citrate, fumerate, tartrate, mesylate and succinate, are suitable, and preferred, for use in the present invention.

In the present invention, "citrate anions" means the deprotonated form of 2-hydroxypropane-1,2,3-trioic acid, commonly known as citric acid. Citrate anions can be provided by 2-hydroxypropane-1,2,3-trioic acid or a pharmaceutically acceptable inorganic salt thereof. Suitable inorganic salts are, for example sodium citrate, and potassium citrate. Preferably, the pharmaceutically acceptable citrate source is citric acid as this does not introduce inorganic cations into the composition of the present invention and citric acid provides not only a suitable counter ion but also provides a means of adjusting the pH to the desired value.

In a preferred embodiment, maropitant is provided by maropitant free base and citrate anions are provided by citric acid. Maropitant free base is only soluble in organic solvents such as DMSO. The inventors have found, however, that an aqueous solution of maropitant can be prepared by addition of citric acid and a β-cyclodextrin. The advantage of this method compared to using maropitant citrate is that maropitant free base is easier to synthesise.

In an embodiment, the maropitant and citrate are provided together by maropitant citrate. For example, maropitant citrate monohydrate may be used which has a molecular weight of 678.81 and a theoretical potency based on the active ingredient of 690 mg/g.

In another embodiment, the pharmaceutically acceptable β-cyclodextrin is sulfobutyl ether 3-cyclodextrin sodium or 2-hydroxypropyl-β-cyclodextrin, preferably sulfobutyl ether β-cyclodextrin. It has been found that sulfobutyl ether β-cyclodextrin is particularly preferred due to the improved solubility of maropitant in the presence of sulfobutyl ether β-cyclodextrin.

In yet another disclosure, the pharmaceutically acceptable preservative is selected from the group consisting of phenylmethanol, 2-phenylethanol, 3-phenyl-1-propanol and 4-phenyl-1-butanol preferably phenylmethanol. The inventors have found that when phenylmethanol is included in the composition according to the present invention, a particularly good injection site tolerance is observed.

In an embodiment, the pharmaceutically acceptable base is sodium hydroxide or ammonia, preferably sodium hydroxide. It has been found that there are no adverse side effects when sodium hydroxide is included in the composition according to the present invention.

In an embodiment, the pharmaceutically acceptable acid is selected from the group consisting of hydrochloric acid, formic acid, acetic acid and citric acid, preferably being citric acid. It has been found that in order to bring the composition to the desirable pH of between 3 and 6, both inorganic acid, for example hydrochloric acid, or organic acids, for example formic acid, acetic acid and citric acid, can be used. Preferably, citric acid is used as this also provides a source of citrate anions, which is the preferred anion in order to achieve the desired injection site tolerance.

The invention will now be illustrated by the following non-limiting examples.

Examples

Materials

Maropitant (free base) was synthesised according to WO 1990005729 A1. Sulfobutyl ether β-cyclodextrin sodium was obtained from Fraupharma (Italy). 2-Hydroxypropyl β-cyclodextrin was obtained from Roquette (France). Phenylmethanol (benzyl alcohol) is obtained from Merck catalogue number 1.00981, 2-phenylethanol, was obtained from Merck, catalogue number 8.07006 and used without any further purification. Methyl parahydroxybenzoate and propyl parahydroxybenzoate were obtained from Merck, catalogue numbers 1.06757 and 1.07427 respectively.

Preparation of formulations

Formulations A1 - A4:

10 mg/ml maropitant free base, 4.1 mg/ml citric acid and cyclodextrin and preservatives as shown in table 1. Formulations were titrated with sodium hydroxide and citric acid to obtain the optimum pH 4.2.

Table 1: Antimicrobial testing of Maropitant formulations A1-A4

| Example | Cyclodextrin | Preservative | Ph. Eur. A criteria | Ph. Eur. B criteria |
|---|---|---|---|---|
| A1 | 63 mg/ml SEB | 10 mg/ml phenylmethanol | Complies | Complies |
| A2 | 63 mg/ml SEB | 1 mg/ml methyl parahyd roxybenzoate 0.1 mg/ml propyl parahyd roxybenzoate | Does not comply | Does not comply |
| A3 | 63 mg/ml SEB | 11 mg/ml phenylmethanol | Complies | Complies |
| A4 | 45 mg/ml HP | 10 mg/ml phenylmethanol | Complies | Complies |
| SEB = sulfobutyl ether β-cydodextrin sodium<br>HP = 2-Hydroxypropyl β-cyclodextrin | | | | |

Antimicrobial efficacy was tested according to European Pharmacopoeia 8.0 chapter 5.1.3, 01/2011:50103. Briefly, the test consists of challenging the formulation with a prescribed inoculum of suitable micro-organisms, storing in inoculated formulation at a prescribed temperature, withdrawing a sample of the formulation at specified time intervals and

counting the organisms in the samples so removed. The acceptance criteria for the injectable formulations tested correspond to those of Table 5.1.3.1 of Eu. Pharm. 8.0.

Osmolality

Osmolality was measured according European Pharmacopoeia 8.0 01/2012 (Eu. Pharm 8.0), chapter 2.2.35. Briefly, reference solutions according to Table 2.2.35-1 of Eu. Pharm 8.0 were prepared. The zero of the apparatus was determined using water. The apparatus was calibrated using the reference solutions. Before testing each sample, the measurement cell was rinsed with the sample to be tested, see table 2.

Table 2: Osmolality of formulations A1 - 4

| Example | Osmality / mOsm/kg |
|---|---|
| A1 | 274 |
| A2 | 42 |
| A3 | 301 |
| A4 | 122 |

Example 5

Injection site tolerance

The formulations given in table 1 were assessed for injection site tolerance as follows. 10 Cats were injected with a formulation according to the following tables 3 - 5. The injection site tolerance was scored on a scale of 1 - 5, where 1 indicates a minimal pain response was observed and 5 indicates severe pain response was observed.

Table 3: Injection site tolerance of commercially available Cerenia® which contains maropitant citrate, sulfobutyl ether β-cyclodextrin sodium & metacresol

| Cerenia | Weight (kg) | Volume of injected fluid(ml) | Scoring (1-2-3-4-5) |
|---|---|---|---|
| Cat 1 | 3,3 | 0,2 | 1 |
| Cat 2 | 5 | 0,5 | 3 |
| Cat 3 | 3,5 | 0,2 | 3 |
| Cat 4 | 3 | 0,2 | 3 |
| Cat 5 | 2,8 | 0,2 | 1 |
| Cat 6 | 6 | 0,4 | 3 |
| Cat 7 | 2,6 | 0,2 | 4 |
| Cat 8 | 4 | 0,2 | 2 |
| Cat 9 | 4 | 0,2 | 2 |
| Cat 10 | 3,5 | 0,2 | 2 |
| MEAN SCORE | | | 2.4 |
| MODE SCORE | | | 3 |

Table 4: Injection site tolerance of test formulation A4 (10 mg/ml maropitant free base, 4.1 mg/ml citric acid, 45 mg/ml 2-Hydroxypropyl β-cyclodextrin, 10 mg/ml phenylmethanol)

| Test formulation 1 | Weight (kg) | Volume of injected fluid(ml) | Scoring (1-2-3-4-5) |
|---|---|---|---|
| Cat 1 | 2,5 | 0,15 | 1 |
| Cat 2 | 2,5 | 0,15 | 2 |
| Cat 3 | 2,7 | 0,15 | 2 |

(continued)

| Test formulation 1 | Weight (kg) | Volume of injected fluid(ml) | Scoring (1-2-3-4-5) |
|---|---|---|---|
| Cat 4 | 4 | 0,2 | 2 |
| Cat 5 | 3 | 0,15 | 1 |
| Cat 6 | 2,9 | 0,15 | 2 |
| Cat 7 | 3 | 0,15 | 2 |
| Cat 8 | 4,9 | 0,25 | 1 |
| Cat 9 | 3 | 0,15 | 3 |
| Cat 10 | 2,6 | 0,13 | 1 |
| MEANS SCORE | | | 1.6 |
| MODE SCORE | | | 2 |

Table 5: Injection site tolerance of test formulation A1 (10 mg/ml maropitant free base, 4.1 mg/ml citric acid, 63 mg/ml sulfobutyl ether β-cyclodextrin sodium, 10 mg/ml phenylmethanol)

| Test formulation 2 | Weight (kg) | Amount injected fluid (ml) | Scoring (1-2-3-4-5) |
|---|---|---|---|
| Cat 1 | 3,5 | 0,2 | 1 |
| Cat 2 | 5,5 | 0,24 | 1 |
| Cat 3 | 4,0 | 0,2 | 1 |
| Cat 4 | 3,5 | 0,17 | 1 |
| Cat 5 | 3 | 0,15 | 2 |
| Cat 6 | 2,9 | 0,15 | 1 |
| Cat 7 | 3,0 | 0,15 | 1 |
| Cat 8 | 3,15 | 0,15 | 1 |
| Cat 9 | 2,8 | 0,14 | 1 |
| Cat 10 | 3 | 0,15 | 3 |
| Cat 11 | 2,5 | 0,12 | 1 |
| MEAN SCORE | | | 1.2 |
| MODE SCORE | | | 1 |

The results show that formulations containing phenylmethanol have a much better injection site tolerance than the commercially available Cerenia. Injection of a formulation comprising 2-hydroxypropyl β-cyclodextrin and phenymethanol (test formulation A4) led to a one point decrease in pain score, with the mode being a score of 2 compared to 3.

Injection of a formulation comprising sulfobutyl ether β-cyclodextrin and phenylmethanol (test formulation A1) instead of sulfobutyl ether β-cyclodextrin and meta-cresol (Cerenia - control) led to a surprising 2 point decrease in pain score, with a mode of 1 compared to 3. Clearly, phenylmethanol provides an injectable composition with improved injection site tolerance compared to the commercially used meta-cresol.

Example 6

Testing of different maropitant citrate formulations. 10 mg/ml maropitant free base corresponds to the same maropitant content as 14.5 mg/ml maropitant citrate monohydrate. Similarly, 4.1 mg/ml citric acid corresponds, in terms of citrate anions, to 4.6 mg/ml sodium citrate.

Formulation series A:

10 mg/ml maropitant free base, 4.1 mg/ml citric acid, 63 mg/ml sulfobutyl ether β-cyclodextrin and preservatives as shown in table 6.

Formulation series B:

14.5 mg/ml maropitant citrate monohydrate, 63 mg/ml sulfobutyl ether β-cyclodextrin and preservatives as shown in table 6.

Formulation series C

10 mg/ml maropitant free base, 4.6 mg/ml sodium citrate, 63 mg/ml sulfobutyl ether β-cyclodextrin, and preservatives as shown in table 6, adjusted to pH 4.4 using hydrochloric acid and sodium hydroxide.

Table 6: Testing of maropitant formulations A, B and C and antimicrobial preservatives

| Example | Maropitant formulation | Antimicrobial preservative | Injection site tolerance |
|---|---|---|---|
| 5.1 | A | 10 mg / ml Phenylmethanol | 1 |
| 5.2 | | 10 mg / ml Phenyl ethanol | 2 |
| 5.3 | | 4.1 mg/ml Meta-cresol | 3 |
| 5.4 | B | 10 mg / ml Phenylmethanol | 1 |
| 5.5 | | 10 mg / ml Phenyl ethanol | 2 |
| 5.6 | | 4.1 mg/ml Meta-cresol | 3 |
| 5.7 | C | 10 mg / ml Phenylmethanol | 1 |
| 5.8 | | 10 mg / ml Phenyl ethanol | 2 |
| 5.9 | | 4.1 mg/ml Meta-cresol | 3 |

As can be seen, an acceptable injection site tolerance (1) is obtained when maropitant citrate is provided by either maropitant free base and citric acid (A), maropitant citrate (B) or maropitant free base and sodium citrate (C). The component effecting injection site tolerance is the antimicrobial preservative. When meta-cresol is used a poor injection site tolerance is obtained (3), whereas phenylethanol and phenylmethanol have good (2) and very good (1) tolerances respectively. When 1 mg/ml methyl parahydroxybenzoate together with 0.1 mg/ml propyl parahydroxybenzoate was used as preservative (not shown) the desired level of antimicrobial efficacy was not obtained.

The results show that compounds corresponding to general Formula II, exhibit the desired injection site tolerance and antimicrobial efficacy, whereas those compounds not falling within the scope of general Formula II, do not show the desired injection site tolerance and antimicrobial efficacy.

## Claims

**1.** Pharmaceutical composition comprising:

an aqueous solution of:

a) maropitant citrate, the maropitant having the structural formula I:

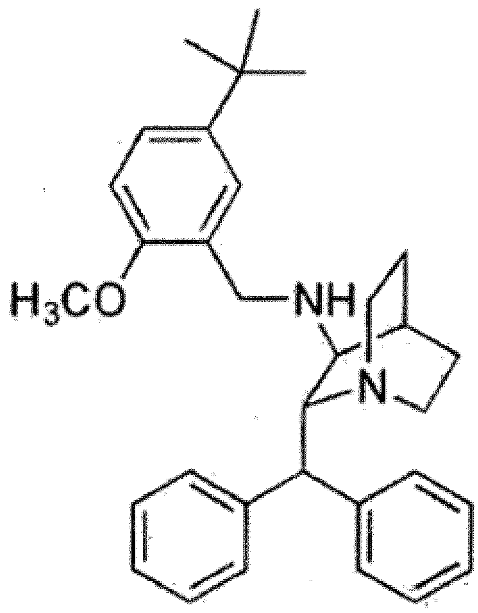

Formula I

b) a pharmaceutically acceptable β-cyclodextrin,
c) 7 - 18 mg/ml of a pharmaceutically acceptable preservative according to formula II:

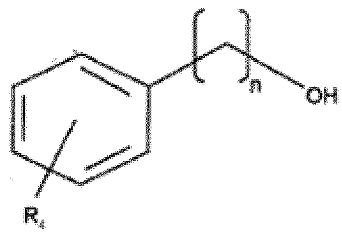

Formula II

wherein $R_x$ is selected from the group consisting of hydrogen, $C_1$-$C_5$ hydrocarbyl, heteroatom or halogen substituted $C_1$-$C_5$ hydrocarbyl, halogens and heteroatoms, x being an integer between 1 and 5, n being an integer between 1 and 4,
wherein the pharmaceutically acceptable preservative is phenylmethanol.

2. Pharmaceutical composition according to claim 1, wherein the pharmaceutically acceptable preservative is present at a concentration of 8-12 mg/ml, preferably 9 - 11 mg/ml, most preferably about 10 mg/ml.

3. Pharmaceutical composition according to any of the preceding claims, wherein the preservative is free of free of chlorobutanol, preferably free of chlorinated compounds, most preferably the aqueous solution being free of chlorinated organic compounds.

4. Pharmaceutical composition according any of the preceding claims, comprising 8-12 mg/ml maropitant, as calculated by the weight of the maropitant free base.

5. Pharmaceutical composition according to any of the previous claims, comprising 40 - 80, preferably 60 - 70 mg/ml of the pharmaceutically acceptable β-cyclodextrin.

6. Pharmaceutical composition according to any of the preceding claims, wherein the pharmaceutically acceptable β-cyclodextrin is sulfobutyl ether β-cyclodextrin sodium or 2-hydroxypropyl-β-cyclodextrin, preferably sulfobutyl ether β-cyclodextrin.

**7.** Pharmaceutical composition according to any of the previous claims, wherein the osmolality of the solution is between 290 and 310 mOsm/kg.

**8.** Pharmaceutical composition according to any of the previous claims, having a pH of 3 - 6.

**9.** Pharmaceutical composition according to any one or more of the previous claims for use as a medicament.

**10.** Pharmaceutical composition according to any one or more of claims 1-8, for use in the treatment and prevention of emesis in mammals, in particular cats and dogs.

**11.** Pharmaceutical composition for use according to claim 9 or 10, wherein said composition is administered by parenteral injection.

**12.** Method for preparing a pharmaceutical composition according to any one or more of claims 1-8, comprising the steps of:

I) mixing, in an aqueous medium, the medium preferably being water:

i) a source of maropitant,
ii) a source of citrate,
iii) a pharmaceutically acceptable β-cyclodextrin to the mixture, the pharmaceutically acceptable β-cyclodextrin preferably being sulfobutyl ether β-cyclodextrin sodium or 2-hydroxypropyl-β-cyclodextrin, most preferably sulfobutyl ether β-cyclodextrin,
iv) a pharmaceutically acceptable preservative as defined in claim 1, to obtain an aqueous solution,

II) if the pH of the aqueous solution is outside the range of 3 - 6, adjusting the pH to between 3 and 6 by adding to the solution a pharmaceutically acceptable acid or base, the pharmaceutically acceptable base preferably being sodium hydroxide or ammonia, most preferably sodium hydroxide, the pharmaceutically acceptable acid preferably being selected from the group consisting of hydrochloric acid, formic acid, acetic acid and citric acid, preferably being citric acid.

**13.** Method according to claim 12, wherein the source of maropitant is selected from the group, consisting of maropitant free base or a maropitant salt, said salt preferably being selected from the group consisting of hydrobromide, hydrochloride, hydroiodide, sulphate, acetate, malate, oxalate, lactate, fumerate, tartrate, mesylate and succinate, the source preferably being maropitant free base, the source of citrate being selected from the group consisting of sodium citrate, potassium citrate and citric acid, preferably being citric acid, or wherein the source of both maropitant and citrate is maropitant citrate.

## Patentansprüche

**1.** Pharmazeutische Zusammensetzung, die Folgendes umfasst:

eine wässrige Lösung von:

a) Maropitantcitrat, wobei das Maropitant die Strukturformel I aufweist:

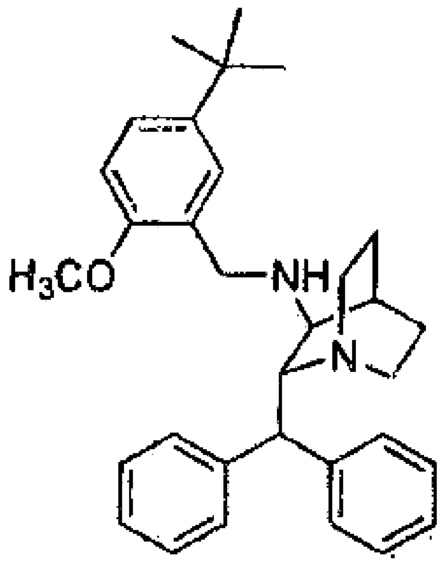

Formel I

b) einem pharmazeutisch annehmbaren β-Cyclodextrin,
c) 7-18 mg/ml eines pharmazeutisch annehmbaren Konservierungsmittels der Formel II:

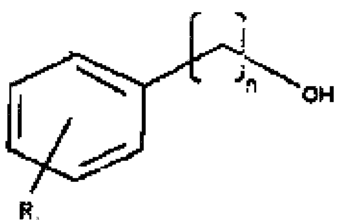

worin $R_x$ aus der aus Wasserstoff, $C_{1-5}$-Hydrocarbyl, mit einem Heteroatom oder Halogen substituierten $C_{1-5}$-Hydrocarbyl, Halogenen und Heteroatomen bestehenden Gruppe ausgewählt ist, wobei x eine ganze Zahl zwischen 1 und 5 ist und n eine ganze Zahl zwischen 1 und 4 ist,
wobei das pharmazeutisch annehmbare Konservierungsmittel Phenylmethanol ist.

**2.** Pharmazeutische Zusammensetzung nach Anspruch 1, in der das pharmazeutisch annehmbare Konservierungsmittel in einer Konzentration von 8-12 mg/ml, vorzugsweise 9-11 mg/ml, am meisten bevorzugt etwa 10 mg/ml, vorhanden ist.

**3.** Pharmazeutische Zusammensetzung nach einem der vorangegangenen Ansprüche, wobei das Konservierungsmittel frei von Chlorbutanol, vorzugsweise frei von chlorierten Verbindungen, ist, wobei die wässrige Lösung am meisten bevorzugt frei von chlorierten organischen Verbindungen ist.

**4.** Pharmazeutische Zusammensetzung nach einem der vorangegangenen Ansprüche, die 8-12 mg/ml Maropitant, berechnet als Gewicht der freien Maropitant-Base, umfasst.

**5.** Pharmazeutische Zusammensetzung nach einem der vorangegangenen Ansprüche, die 40-80 mg/ml, vorzugsweise 60-70 mg/ml, des pharmazeutisch annehmbaren β-Cyclodextrins enthält.

**6.** Pharmazeutische Zusammensetzung nach einem der vorangegangenen Ansprüche, wobei das pharmazeutisch annehmbare β-Cyclodextrin Sulfobutylether-β-cyclodextrinnatrium oder 2-Hydroxypropyl-β-cyclodextrin ist, vorzugsweise Sulfobutylether-β-cyclodextrin, ist.

**7.** Pharmazeutische Zusammensetzung nach einem der vorangegangenen Ansprüche, wobei die Osmolalität der Lösung zwischen 290 und 310 mOsm/kg liegt.

**8.** Pharmazeutische Zusammensetzung nach einem der vorangegangenen Ansprüche, die einen pH-Wert von 3-6 aufweist.

**9.** Pharmazeutische Zusammensetzung nach einem oder mehreren der vorangangenen Ansprüche zur Verwendung als Medikament.

**10.** Pharmazeutische Zusammensetzung nach einem oder mehreren der Ansprüche 1 bis 8 zur Verwendung bei der Behandlung und Vorbeugung von Erbrechen bei Säugetieren, insbesondere Katzen und Hunden.

**11.** Pharmazeutische Zusammensetzung zur Verwendung nach Anspruch 9 oder 10, wobei die Zusammensetzung durch parenterale Injektion verabreicht wird.

**12.** Verfahren zur Herstellung einer pharmazeutischen Zusammensetzung nach einem oder mehreren der Ansprüche 1 bis 8, das die folgenden Schritte umfasst:

I) das Vermischen, in einem wässrigen Medium, wobei das Medium vorzugsweise Wasser ist:

i) einer Quelle von Maropitant,
ii) einer Quelle von Citrat,
iii) eines pharmazeutisch annehmbaren β-Cyclodextrins in das Gemisch, wobei das pharmazeutisch annehmbare β-Cyclodextrin vorzugsweise Cyclodextrin Sulfobutylether-β-cyclodextrin-natrium oder 2-Hydroxypropyl-β-cyclodextrin, am meisten bevorzugt Sulfobutylether-β-cyclodextrin, ist,
iv) eines pharmazeutisch annehmbaren Konservierungsmittels wie in Anspruch 1 definiert, um eine wässrige Lösung zu erhalten,

II) falls der pH-Wert der wässrigen Lösung außerhalb des Bereichs von 3-6 liegt, das Einstellen des pH-Werts auf einen Wert zwischen 3 und 6 durch Zusetzen einer pharmazeutisch annehmbaren Säure oder Base zu der Lösung, wobei die pharmazeutisch annehmbare Base vorzugsweise Natriumhydroxid oder Ammoniak, am meisten bevorzugt Natriumhydroxid, ist, wobei die pharmazeutisch annehmbare Säure vorzugsweise aus der aus Salzsäure, Ameisensäure, Essigsäure und Citronensäure bestehenden Gruppe ausgewählt ist und vorzugsweise Citronensäure ist.

**13.** Verfahren nach Anspruch 12, wobei die Maropitant-Quelle aus der aus der freien Maropitant-Base und einem Maropitant-Salz bestehenden Gruppe ausgewählt ist, wobei das Salz vorzugsweise aus der aus Hydrobromid, Hydrochlorid, Hydroiodid, Sulfat, Acetat, Malat, Oxalat, Lactat, Fumarat, Tartrat, Mesylat und Succinat bestehenden Gruppe ausgewählt ist, wobei die Quelle vorzugsweise die freie Base von Maropitant ist, wobei die Quelle von Citrat aus der aus Natriumcitrat, Kaliumcitrat und Citronensäure bestehenden Gruppe ausgewählt ist, wobei sie vorzugsweise Citronensäure ist, oder wobei die Quelle sowohl von Maropitant als auch von Citrat Maropitant-Citrat ist.

## Revendications

**1.** Composition pharmaceutique comprenant :

une solution aqueuse de :

a) de citrate de maropitant, le maropitant ayant la formule structurelle I :

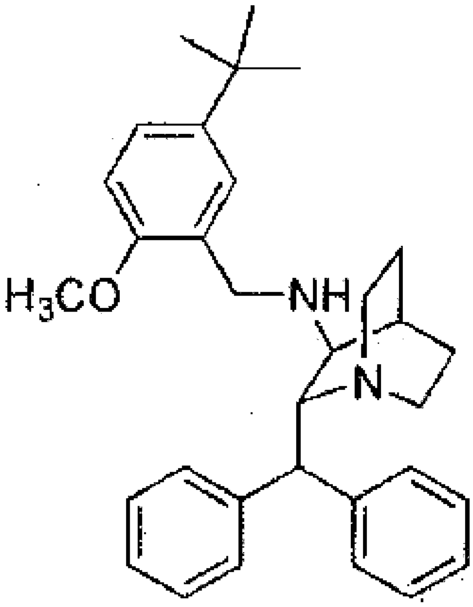

Formule I

b) une β-cyclodextrine pharmaceutiquement acceptable,

c) 7 - 18 mg/ml d'un conservateur pharmaceutiquement acceptable de formule II :

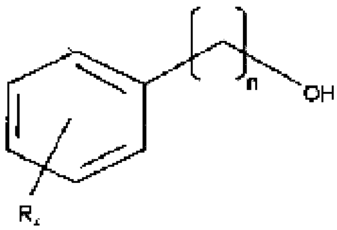

Formule II

dans laquelle $R_x$ est choisi dans le groupe comprenant hydrogène, hydrocarbyle en $C_1$-$C_5$, hétéroatome ou hydrocarbyle en $C_1$-$C_5$ substitué par halogène, halogènes et hétéroatomes, x étant un entier compris entre 1 et 5, et n étant un entier compris entre 1 et 4,

dans laquelle le conservateur pharmaceutiquement acceptable est le phénylméthanol.

**2.** Composition pharmaceutique selon la revendication 1, dans laquelle le conservateur pharmaceutiquement acceptable est présent à une concentration de 8 - 12 mg/ml, de préférence 9 - 11 mg/ml, de préférence environ 10 mg/ml.

**3.** Composition pharmaceutique selon l'une quelconque des revendications précédentes, dans laquelle le conservateur est libre de chlorobutanol, de préférence exempt de composés chlorés, la solution aqueuse étant de la manière la plus préférée libre de composés organiques chlorés.

**4.** Composition pharmaceutique selon l'une quelconque des revendications précédentes, comprenant 8 - 12 mg/ml de maropitant, calculé par le poids de la base libre du maropitant.

**5.** Composition pharmaceutique selon l'une quelconque des revendications précédentes, comprenant 40 - 80, de préférence 60 - 70 mg/ml de la β-cyclodextrine pharmaceutiquement acceptable.

**6.** Composition pharmaceutique selon l'une quelconque des revendications précédentes, dans laquelle la β-cyclodextrine pharmaceutiquement acceptable est l'éther sulfobutylique β-cyclodextrine sodique ou la 2-hydroxypropyl-β-cyclodextrine, de préférence l'éther sulfobutylique β-cyclodextrine.

7. Composition pharmaceutique selon l'une quelconque des revendications précédentes, dans laquelle l'osmolalité de la solution est comprise entre 290 et 310 mOsm/kg.

8. Composition pharmaceutique selon l'une quelconque des revendications précédentes, ayant un pH de 3 à 6.

9. Composition pharmaceutique selon l'une quelconque ou plusieurs des revendications précédentes, pour utilisation comme médicament.

10. Composition pharmaceutique selon l'une ou plusieurs des revendications 1 à 8, destinée à être utilisée dans le traitement et la prévention des vomissements chez les mammifères, en particulier les chats et les chiens.

11. Composition pharmaceutique pour utilisation selon la revendication 9 ou 10, dans laquelle ladite composition est administrée par injection parentérale.

12. Procédé de préparation d'une composition pharmaceutique selon l'une ou plusieurs des revendications 1 à 8, comprenant les étapes suivantes :

I) mélanger, dans un milieu aqueux, le milieu étant de préférence de l'eau :

i) une source de maropitant,
ii) une source de citrate,
iii) une β-cyclodextrine pharmaceutiquement acceptable pour le mélange, la β-cyclodextrine pharmaceutiquement acceptable étant de préférence l'éther sulfobutylique β-cyclodextrine sodique ou la 2-hydroxypropyl-β-cyclodextrine, de la manière la plus préférée l'éther sulfobutylique β-cyclodextrine,
iv) un agent de conservation pharmaceutiquement acceptable tel que défini dans la revendication 1, pour obtenir une solution aqueuse,

II) si le pH de la solution aqueuse est en dehors de la plage de 3 à 6, ajuster le pH entre 3 et 6 en ajoutant à la solution un acide ou une base pharmaceutiquement acceptable, la base pharmaceutiquement acceptable étant de préférence l'hydroxyde de sodium ou l'ammoniac, de la manière la plus préférée l'hydroxyde de sodium, l'acide pharmaceutiquement acceptable étant de préférence choisi dans le groupe constitué par l'acide chlorhydrique, l'acide formique, l'acide acétique et l'acide citrique, de préférence l'acide citrique.

13. Procédé selon la revendication 12, dans lequel la source de maropitant est choisie dans le groupe constitué par une base libre de maropitant ou un sel de maropitant, ledit sel étant de préférence choisi dans le groupe constitué par le bromure d'hydrogène, le chlorhydrate, l'iodhydrate, le sulfate, l'acétate, le malate, l'oxalate, le lactate, le fumarate, tartrate, mésylate et succinate, la source étant de préférence la base libre de maropitant, la source du citrate étant choisie dans le groupe constitué par le citrate de sodium, le citrate de potassium et l'acide citrique, étant de préférence l'acide citrique, ou dans lequel la source du maropitant et du citrate est le citrate du maropitant.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2005082416 A
- WO 1990005729 A1

### Non-patent literature cited in the description

- **LUI, RONG**. Water-insoluble Drug Formulation. CRC Press, 2008, 180
- Pharmacopoeia. 01 December 2009, S5-38